# EUROPEAN PATENT APPLICATION

(11) **EP 1 832 238 A2**
(43) Date of publication of application: **12.09.2007**
(21) Application number: 07103580.2
(22) Date of filing: 06.03.2007
(51) Int. Cl.: A61B 17/06, A61L 17/00

(54) **TAPERED SUTURE**

(30) Priority: 07.03.2006 US 779458 P
(71) Applicant: Arthrex, Inc., Naples, Florida 34108-1945 (US)
(72) Inventor: Dreyfuss, Peter, Naples, FL 34112 (US)
(74) Representative: Holmberg, Martin Tor

(57) **Abstract**

A tapered suture having a core region of a first diameter and at least one tail region of a second diameter, the second diameter being smaller than the first diameter. The suture is made from a material having high strength, and the tapered ends facilitate loading of the suture into instruments intra-operatively.

## Description

### FIELD OF THE INVENTION

The present invention relates to surgical sutures and, more particularly, to a tapered suture.

### BACKGROUND OF THE INVENTION

In endoscopic surgical procedures, suturing internal body tissue presents particularly challenging tasks. In such minimally invasive type surgical procedures, suturing must be accomplished with suture passing or retrieving instruments that typically average only few millimeters in diameter. Loading of the suture onto various surgical instruments intra-operatively is also complex and requires cannulated inserters and instruments having a specific configuration and diameter to avoid trauma to the adjacent tissue.

The ideal suture for use in surgery would be one that is strong, easy for a surgeon to handle and knot reliably, and allow loading the suture into instruments intra-operatively.

Suture strands used in conjunction with suture anchors, such as threaded suture anchors, for example, are increasingly used during surgical procedures when tissue tears away from the bone. A cannulated inserter for a suture anchor has a relatively small inner diameter. On most anchors, two sutures are loaded on the anchor, creating four suture tails that must come up through the cannulation of the inserter. In a production setting, which pristine sutures, it is not difficult to advance the ends of the four suture limbs straight through the cannulation of the inserter. In a clinical setting, however, with some passing of those sutures through tissue already possibly having occurred during surgery, the sutures are wet, and "bunched" or otherwise, and it becomes extremely difficult, if not impossible, to push them through the cannulation of an inserter.

Due to the relatively small size of the inner diameter of the inserter, using a suture passing wire (a small nitinol wire with a very small nitinol loop at one end) to pass the suture tails is impossible because the sutures must be doubled over when using the passing wire - eight limbs of suture will not fit within the cannulation of an inserter.

Accordingly, it would be desirable to provide a suture which can be easily loaded, intra-operatively, into instruments, and which has other advantages.

### SUMMARY OF THE INVENTION

The present invention fulfills the needs noted above by providing a tapered suture having a core region of a first diameter and at least one tail region of a second diameter, the second diameter being smaller than the first diameter.
Preferably, the tapered suture has two opposing tail regions having similar or different diameters, but having diameters smaller than the diameter of the core region. Because of the thin tail regions, the tapered suture of the present invention may be employed in conjunction with suture passing and retrieving instruments that require a diameter smaller than that of the instruments of the prior art. In addition, the tapered suture is more easily threaded through instruments, such as a suture anchor inserter.

The tapered suture of the present invention may comprise a high strength suture material with surgically-useful qualities, including knot tie down characteristics and handling. Alternatively or additionally, the tapered suture of the present invention may be formed of a bioabsorbable material, such as PLLA for example, may be coated or uncoated, and may include twisted or braided threads optionally including colored strands such as dyed filaments.

These and other features and advantages of the present invention will become apparent from the following description of the invention that is provided in connection with the accompanying drawings and illustrated embodiments of the invention.

### BRIEF DESCRIPTION OF THE DRAWING

FIG. 1 illustrates a first embodiment of a tapered suture of the present invention; and

FIG. 2 illustrates a second embodiment of a tapered suture of the present invention.

### DETAILED DESCRIPTION OF THE INVENTION

The present invention is a tapered suture having a core region of a first diameter and at least one tail region of a second diameter, the second diameter being smaller than the first diameter; the tail region having high strength to facilitate loading of the suture into instruments intra-operatively.

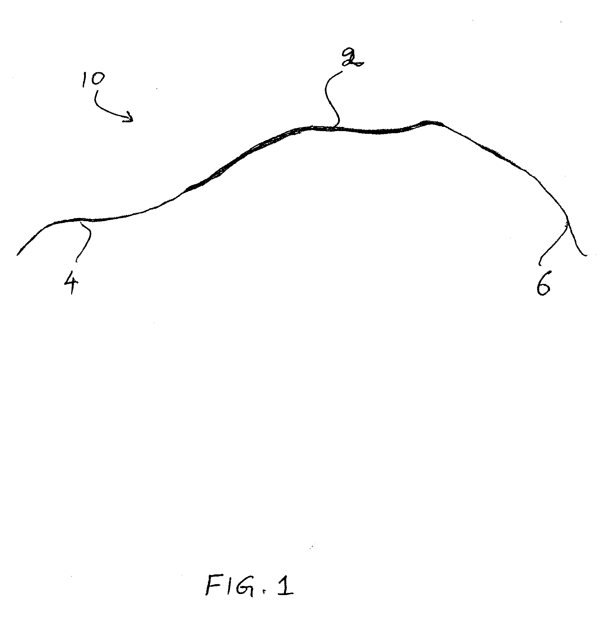

Referring now to the drawing, where like elements are designated by like reference numerals, FIG. 1 illustrates a first embodiment of a tapered suture according to the present invention. The tapered suture **10** is formed of a core region **2** which adjacent two tail regions **4** and **6.** As shown in FIG. 1, the diameter of the tail region **4** may be similar to or different than the diameter of the tail region **6.** In any event, each of the diameters of the tail regions **4, 6** is smaller than the diameter of the core region **2.** In a preferred embodiment, the tail regions **4, 6** are #0 sutures, and the core region **2** is a #2 suture.

As a result of the smaller diameter of the tail regions **4, 6,** the tapered suture **10** of the present invention is more easily threaded through the eyelet of a suture anchor or a suture anchor inserter, for example. The tapered suture **10** of the present invention can also be readily threaded through needles having a small eyelet, which in turn advantageously make a small hole in tissue.

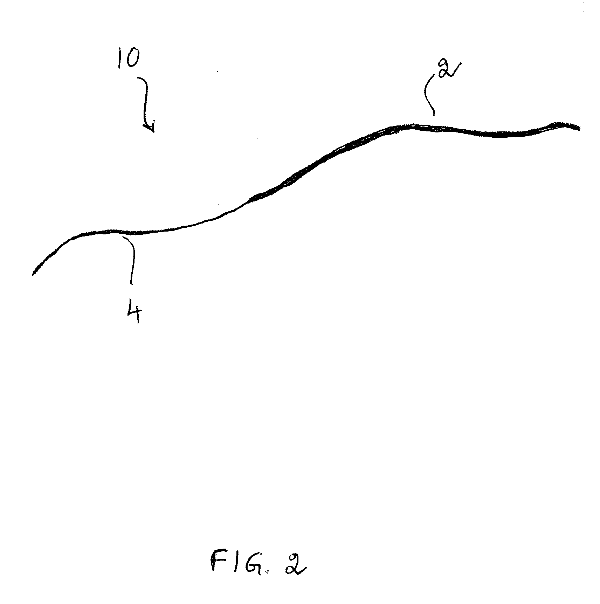

Referring to FIG. 2, which illustrates a second embodiment of a tapered suture of the present invention, the tapered suture **10** is formed of a core region **2** with a tail region **4.** The diameter of the tail region **4** is smaller than the diameter of the core region **2.** In a preferred embodiment, the tail region **4** is a #0 suture, and the core region **2** is a #2 suture.

The tapered suture **10,** shown in FIGS. 1 and 2, may be used in an instrument for arthroscopic suture passing, as is described, for example, in U.S. Patent No. 6,896,686 ("Weber"), assigned to Arthrex, Inc. By using the tapered suture **10** in an instrument as described in Weber, the instrument may be used with a smaller needle resulting in a smaller hole in tissue and thereby, reducing the chances of the suture snagging when the instrument is retrieved.

The tapered suture **10** may contain strands of a high strength suture material, such as Arthrex FiberWire^{®} suture disclosed in U.S. Pat. No. 6,716,234, with optional colored strands (preferably black) to assist surgeons in distinguishing between suture lengths with the trace and suture lengths without the trace.

The tapered suture **10** is preferably coated (partially or totally) with wax (beeswax, petroleum wax, polyethylene wax, or others), silicone (Dow Corning silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the braid, knot security, pliability, handleability or abrasion resistance, for example.

Tapered suture **10** may also contain a bioabsorbable material, such as PLLA or one of the other polylactides, for example, and/or may be formed of twisted fibers having strands of a contrasting color added to the braided threads, to make the suture more visible during surgical procedures. The colored strands, preferably, may be dyed filaments or strands.

In one embodiment, the tail regions **4, 6** of the tapered suture 10 are provided with tinted tracing strands, or otherwise contrast visually with the core region of the tapered suture, which remains a plain, solid color, or displays a different tracing pattern, for example. Accordingly, when the tapered suture is loaded through the eyelet of a suture anchor or passed through tissue, for example, at least one of the tail regions **4, 6** of the suture is visually coded, making identification and handling of the suture legs simpler. Easy identification of suture *in situ* is advantageous in surgical procedures, particularly during arthroscopic surgeries, such as endoscopy and laparoscopy.

In yet another embodiment, the tail regions **4, 6** may have very fine ends that are coated, impregnated or otherwise stiffened with plastic.

The tapered suture **10** of the present invention may be employed in surgical procedures such as rotator cuff repair, Achilles tendon repair, patellar tendon repair, ACL/PCL reconstruction, hip and shoulder reconstruction procedures, and replacement for suture used in or with suture anchors.

While the present invention is described herein with reference to illustrative embodiments for particular applications, it should be understood that the invention is not limited thereto. Those having ordinary skill in the art and access to the teachings provided herein will recognize additional modifications, applications, embodiments and substitution of equivalents all fall within the scope of the invention. Accordingly, the invention is not to be considered as limited by the foregoing description.

## Claims

1. A suture strand suitable for use as a suture or ligature, comprising:
a first portion of a first diameter; and
at least one second portion of a second diameter, wherein the second portion is at an end region of the suture strand.

2. The suture strand of claim 1, wherein the first diameter is greater than the second diameter.

3. The suture strand of claim 1, wherein the suture strand is formed of a strand material comprising a high strength material.

4. The suture strand of claim 3, wherein the strand material further comprises a bioabsorbable material.

5. The suture strand of claim 4, wherein the bioabsorbable material comprises poly-L-lactide, and poly-DL-lactide.

6. The suture strand of claim 3, wherein the strand material is coated with a material comprising wax, silicone, polytetrafluoroethylene, and polybutylate acid.

7. The suture strand of claim 1, wherein the second portion has an end coated with a plastic material.

8. The suture strand of claim 1, wherein the second portion has an end impregnated with a plastic material.
